# EUROPÄISCHE PATENTSCHRIFT

(11) **EP 0 704 206 B1**
(45) Veröffentlichungstag und Bekanntmachung des Hinweises auf die Patenterteilung: **04.09.2002**
(21) Anmeldenummer: 95115315.4
(22) Anmeldetag: 28.09.1995
(51) Int. Cl.: A61K 9/12, A61K 9/127, A61K 45/08, A61K 31/19, A61K 31/60, A61K 31/485, A61K 31/715, A61K 31/57, A61K 31/165, A61K 31/49, A61K 38/22, A61K 31/52, A61K 31/557

(54) **Pharmazeutische Zusammensetzung**
Pharmaceutical composition
Composition pharmaceutique

(30) Priorität: 30.09.1994 DE 4434995; 30.09.1994 DE 4435010
(43) Veröffentlichungstag der Anmeldung: 03.04.1996
(73) Patentinhaber: MIKA Pharma Gesellschaft für die Entwicklung und Vermarktung pharmazeutischer Produkte mbH, 67117 Limburgerhof (DE)
(72) Erfinder: Regenold, Jürgen, Dr., D-79285 Ebringen (DE); Artmann, Carl, D-82131 Gauting (DE); Röding, Joachim, Dr., D-65207 Wiesbaden (DE)
(74) Vertreter: Lau-Loskill, Philipp, Dipl.-Phys.

(56) Entgegenhaltungen:
- EP-A- 0 103 783
- EP-A- 0 130 550
- EP-A- 0 253 619
- EP-A- 0 267 050
- EP-A- 0 292 100
- EP-A- 0 391 342
- EP-A- 0 439 042
- WO-A-87/07504
- WO-A-88/04556
- WO-A-91/07947
- WO-A-94/05330
- DE-A- 4 121 389
- DE-A- 4 122 661
- DE-A- 4 216 644
- US-A- 3 592 891
- US-A- 4 464 378
- US-A- 5 192 528
- DUBBEL, Taschenbuch für den Maschinenbau, Berichtigter Neudruck der 13. Auflage, 1. Band, Sass F.,Bouché ch. und Leitner A. (Hrsgb.), Springer-Verlag Berlin Heidelberg New York 1974, S.876-877
- Römpp Chemielexikon, 9. Auflage, S. 2984, 4937-4940

## Beschreibung

Die vorliegende Erfindung betrifft eine pharmazeutische Zusammensetzung mit den Merkmalen des Oberbegriffs des Patentanspruchs 1.

Pharmazeutische Zusammensetzungen mit dem im Hauptanspruch genannten Wirkstoffen werden üblicherweise als injizierbare Zusammensetzungen oder in Form von Tabletten, Dragees oder Zäpfchen formuliert, um so sicherzustellen, daß innerhalb von kürzester Zeit der jeweilige Wirkstoff über die entsprechende Körperflüssigkeit, insbesondere über das Blut, zum Wirkort transportiert wird, um so systemisch die jeweilige erwünschte Wirkung herbeizuführen.

Nur in wenigen Ausnahmefällen werden die im Hauptanspruch aufgeführten Wirkstoffe so formuliert, daß sie topisch anwendbar sind, wobei eine derartige Anwendung dann vorzugsweise für eine lokale Applikation, d.h. zur örtlich begrenzten äußerlichen Behandlung, geeignet ist. So sind beispielsweise Rheumasalben mit nichtsteroidalen Antirheumatika oder Heparin-haltigen Salben oder Cremes bekannt, die jedoch rein lokal auf die erkrankten Körperbereich aufgetragen und dort regional begrenzt wirksam werden.

Hierzu ist es erforderlich, daß diese Salben oder Cremes auf den regional begrenzten, erkrankten oder mit Beschwerden behafteten Körperbereich aufgetragen und verrieben werden, was insbesondere bei besonders druckempfindlichen lokal begrenzten Erkrankungen oder Verletzungen vielfach als schmerzhaft empfunden wird.

Ebenfalls sind topisch applizierbare Zusammensetzungen bekannt, die lokal aufgesprüht werden. So beschreibt beispielsweise die EP 0 103 783 eine derartige sprühbare Formulierung, die auf die Schleimhaut des Mundes, der Nase oder des Rachens aufgesprüht wird, wobei die sprühbare Formulierung ein Celluloseätherderivat in einer Konzentration zwischen 0,1 und 2 Vol.% als Gelbildner enthält. Weitere, sprühbare Zusammensetzungen mit anderen, nicht phospholipidischen Gelbildnern sind beispielsweise aus der WO 94/05330 A oder der EP 0 391 342 A bekannt.

Phospholipidische Zusammensetzungen, die topisch applizierbar und zusätzlich noch sprühbar sind, sind insbesondere in der US 5,192,528 A, der DE 42 16 644 A und der EP 0 267 050 A desweiteren beschrieben. Hierbei enthalten jedoch diese bekannten Zusammensetzungen die diesbezüglichen Phospholipide nicht als Gelbildner sondern ausschließlich zur Ausbildung von Liposomen definierter Größen, die dann mit dem jeweiligen Wirkstoff als gefüllte, kugelförmige Vesikel vorliegen. Hierbei nennt die US 5,192,528 A einen oberen Wert für den mittleren Liposomendurchmesser von maximal 10 µm, die DE 42 16 644 A einen mittleren Liposomendurchmesser zwischen 100 nm und 200 nm und die EP 0 267 050 A einen mittleren Liposomendurchmesser kleiner als 5 µm, wobei die EP 0 267 050 A explizit herausstellt, daß dann negative Ergebnisse, so zum Beispiel eine toxische Plasmakonzentration an Wirkstoffen, herbeigeführt werden, wenn die versprühten Partikel eine Größenverteilung zwischen 1 µm und 10 µm aufweisen.

Die eingangs genannten, systemisch wirkenden Tabletten, Dragees, Zäpfchen, Injektionen oder Infusionen bereiten insbesondere dann erhebliche Probleme, wenn Patienten über einen längeren Zeitraum behandelt werden müssen. Derartige Probleme, die nicht mit dem Wirkstoff selbst sondern mit der zuvor beschriebenen bekannten Darreichungsform zusammenhängen, drücken sich beispielsweise darin aus, daß Magenschmerzen, Reizungen des Afters und/oder des Darmes oder bei Infusionen oder Injektionen Blutergüsse oder eine Verödung von Venen auftreten, die teilweise so weit führen, daß die Darreichungsform gewechselt oder sogar auf die Gabe des jeweiligen Wirkstoffes verzichtet werden muß.

Der vorliegenden Erfindung liegt die Aufgabe zugrunde, eine pharmazeutische Zusammensetzung der angegebenen Art zur Verfügung zu stellen, die besonders einfach und unproblematisch sowohl lokal als auch systemisch wirksam und topisch applizierbar ist und eine besonders hohe Wirksamkeit aufweist.

Diese Aufgabe wird erfindungsgemäß durch eine pharmazeutische Zusammensetzung mit den kennzeichnenden Merkmalen des Patentanspruchs 1 gelöst.

Die erfindungsgemäße pharmazeutische Zusammensetzung mit mindestens einem systemisch und/oder lokal wirkenden, topisch applizierbaren Wirkstoff, vorzugsweise ausgewählt aus der Gruppe, bestehend aus den Lokalanästhetika, Antiallergika, Dermatika, Wirkstoffen gegen grippale Infekte und Erkältungskrankheiten, Wirkstoffen zur Behandlung von Neuropathien, Chemotherapeutika, Chinin, Thalidomid, Analgetika, nichtsteroidalen Antirheumatika, Opiatrezeptor-Agonisten, Opiatrezeptor-Antagonisten, Blutgerinnung hemmenden Stoffen, Thrombozytenaggregation hemmenden Stoffen, Kortikoiden, Histaminantagonisten, Antidiabetika, regulatorischen Peptiden und ihren Hemmstoffen, Prostaglandinen und/oder ihren Estern und/oder antiviral wirksamen Stoffen, weist eine solche flüssige Konsistenz auf, daß sie als Tröpfchen auf eine Körperfläche versprühbar ist.

Hierbei enthält die flüssige erfindungsgemäße Zusammensetzung einen Gelbildner auf der Basis eines Phospholipids oder eines Phospholipidgemisches in einer Konzentration zwischen 0,5 Gew.% und 20 Gew.%, bezogen auf die anwendungsfertige erfindungsgemäße Zusammensetzung. Desweiteren ist die erfindungsgemäße Zusammensetzung als Tröpfchen mit einem Tröpfchendurchmesser zwischen 10 µm und 100 µm versprühbar, wobei die so versprühte Zusammensetzung nach dem Versprühen innerhalb kürzester Zeit, vorzugsweise innerhalb einer Zeit von weniger als 4 Sekunden, auf der besprühten Körperfläche, insbesondere auf der besprühten Haut oder Schleimhaut, eine solche Zubereitung ausbildet, in der der Wirkstoff dann in einer Feinverteilung vorliegt und in der die Konzentration des mindestens einen Wirkstoffes zwischen 25 % und 150 % höher ist als die Konzentration des mindestens einen Wirkstoffes in der ursprünglichen flüssigen erfindungsgemäßen Zusammensetzung vor dem Versprühen.

Es konnte überraschend festgestellt werden, daß der Zusatz des mindestens einen phospholipidischen Gelbildners die Ausbildung der nebelartigen Tröpfchen beim Versprühen der erfindungsgemäßen flüssigen Zusammensetzung fördert und desweiteren bewirkt, daß die Zusammensetzung nach dem Versprühen innerhalb kürzester Zeit, insbesondere innerhalb der vorstehend genannten Zeiten, auf der besprühten Oberfläche eine gelartige Zubereitung ausbildet. Somit wird auf der besprühten Körperfläche eine gelartige Zubereitung ausgebildet, die den mindestens einen Wirkstoff, den mindestens einen phospholipidischen Gelbildner sowie möglicherweise noch gewisse Anteile der in der ursprünglichen flüssigen Zusammensetzung enthaltenen Flüssigkeiten beinhaltet. Hierdurch wird die Zubereitung und damit auch der mindestens eine Wirkstoff besonders sicher und fest auf der jeweils besprühten Körperfläche, insbesondere der besprühten Haut oder Schleimhaut, fixiert, so daß dann auf dieser Körperfläche ein Depot des mindestens einen Wirkstoffs ausgebildet wird, das sicherstellt, daß ständig Wirkstoff der Körperflüssigkeit, insbesondere dem Blut, und/oder der lokal begrenzten Erkrankung und/oder Verletzung nachgeführt wird.

Zur Vermeidung von Verwirrungen und zur Erleichterung des Verständnisses wird in der vorliegenden Beschreibung die tröpfchenartig versprühbare Darreichungsform, d.h. somit die Darreichungsform, die im Handel erhältlich ist, als erfindungsgemäße pharmazeutische Zusammensetzung bezeichnet, während durchgehend die Form, die sich aus der versprühten erfindungsgemäßen Zusammensetzung durch Verdunsten oder Verdampfen der in der flüssigen Zusammensetzung enthaltenen Flüssigkeit auf der besprühten Körperfläche, insbesondere auf der besprühten Haut und/oder Schleimhaut, ausbildet, mit dem Begriff Zubereitung benannt ist.

Die erfindungsgemäß Zusammensetzung weist eine Reihe von Vorteilen auf. So ist zunächst festzuhalten, daß die erfindungsgemäße Zusammensetzung aufgrund ihrer flüssigen Formulierung wesentlich einfacher und genauer im Vergleich zu den salbenartigen oder cremeartigen bekannten Produkten selbst dort applizierbar ist, wo die bekannten Cremes oder Salben nicht anwendbar sind, so beispielsweise im Bereich der Schläfen, da es bei der flüssigen erfindungsgemäßen Zusammensetzung lediglich erforderlich ist, ein vorgegebenes Volumen zu versprühen, während die salbenartigen bzw. cremeartigen bekannten Produkte eine im wesentlichen gut zugängliche und unbehaarte Hautpartie und ein visuelles Abschätzen der entnommenen Crememenge oder des entnommenen Salbenstrangs erfordern. Auch kann es bei der erfindungsgemäßen flüssigen Zusammensetzung nicht so leicht zu einem Verschmieren oder Verschmutzen von Kleidungsstücken kommen, da ein gerichtetes Aussprühen wesentlich einfacher auch an schwer zugänglichen Körperflächen möglich ist als ein großflächiges Verteilen und Einreiben der bekannten Salben oder Cremes. Desweiteren kann bei der erfindungsgemäßen Zusammensetzung gerade dieses, bei den bekannten Produkten stets erforderliche Verteilen oder Einreiben entfallen, da die auf der Körperfläche durch Verdunsten oder Verdampfen der Flüssigkeit ausgebildete Zubereitung bereits eine ideale Feinstverteilung der Wirkstoffe besitzt, so daß bei der topischen systemischen und/oder lokalen Applikation der erfindungsgemäßen Zusammensetzung dementsprechend auch kein Druckschmerz ausgelöst wird. Darüber hinaus wird von vielen Patienten die durch das Verdunsten bzw. Verdampfen der Flüssigkeit aus der erfindungsgemäßen Zusammensetzung hervorgerufene Kühlung als besonders angenehm und schmerzlindernd empfunden, wodurch die erfindungsgemäße Zusammensetzung schon bei der ersten topischen Applikation beim Patienten den Eindruck hervorruft, daß eine sofortige Heilung eingeleitet wird.

Darüber hinaus sind mit der topischen lokalen und/oder systemischen Applikation der erfindungsgemäßen Zusammensetzung weitere Vorteile verbunden. Bedingt dadurch, daß während oder nach dem Auftragen der flüssigen erfindungsgemäßen Zusammensetzung durch Verdunsten oder Verdampfen der Flüssigkeit eine Zubereitung ausgebildet wird, in der im Vergleich zur ursprünglich eingesetzten flüssigen Zusammensetzung der mindestens eine Wirkstoff in einer wesentlich höheren Konzentration vorliegt, kann, falls erwünscht oder erforderlich, bei der Herstellung der erfindungsgemäßen flüssigen Zusammensetzung darauf verzichtet werden, besonders hohe Konzentrationen des mindestens einen Wirkstoffes mit entsprechend hohem Aufwand und/oder unter Zusatz von unnötigen Zusatzstoffen in der erfindungsgemäßen flüssigen Zusammensetzung zu lösen, dispergieren oder emulgieren, wodurch die Herstellung der erfindungsgemäßen Zusammensetzung vereinfacht wird. Dies ist insbesondere für solche Wirkstoffe wichtig, die nur durch Zusatz von geeigneten Emulgier- bzw. Dispergiermittel in der jeweiligen Flüssigkeit zu emulgieren bzw. dispergieren sind, wie dies beispielsweise auf gewisse injizierbare oder über eine Infusion applizierbare Wirkstoffe oder auf Wirkstoffe auf der Basis von Heparin zutrifft. Auch liegt bei der erfindungsgemäßen flüssigen Zusammensetzung der mindestens eine Wirkstoff nach dem tropfenartigen Aufsprühen der flüssigen Zusammensetzung in der dann auf der jeweiligen Körperfläche ausgebildete Zubereitung in einer idealen Feinstverteilung vor, so daß dieser feinstverteilte Wirkstoff eine extrem kleine Partikelgröße aufweist, was wiederum den Transport des Wirkstoffes, beispielsweise durch Hautbarrieren oder Gewebezellwänden, erleichtert und somit in die entsprechende Körperflüssigkeit, vorzugsweise ins Blut, gelangt und damit zum Wirkort transportiert wird und von daher eine derartige systemische Heilung bewirkt. Aufgrund dieser Feinstverteilung des Wirkstoffes besitzt die erfindungsgemäße Zusammensetzung bei einer lokalen Anwendung eine hohe pharmazeutische Wirksamkeit, so daß erklärlich wird, daß die erfindungsgemäße Zusammensetzung bei einer Anwendung im Bereich der lokalen Therapie wesentlich schneller zur Heilung führt als die herkömmlich und bekannten lokal anzuwendenden Salben und Cremes.

Ein weiterer wesentlicher Vorteil der erfindungsgemäßen Zusammensetzung ist darin zu sehen, daß im einfachsten Fall der mindestens eine Wirkstoff zusammen mit dem phospholipidischen Gelbildner nach dem Verdampfen oder Verdunstender Flüssigkeit in der dadurch ausgebildeten Zubereitung ohne jegliche weitere Hilfsstoffe auf der jeweiligen Körperfläche, insbesondere auf der besprühten Haut oder Schleimhaut, vorliegt, so daß dann dieser mindestens eine Wirkstoff zwangsläufig ohne negative Beeinflussung durch solche Hilfsstoffe, die in den bekannten Salben oder Cremes zwangsläufig enthalten sind, in den Körper diffundiert oder migriert, was bei den bekannten cremeartigen oder salbenartigen Zusammensetzungen nicht der Fall ist. Hierbei wird häufig beobachtet, daß z.B. der Salbengrundstoff oder der Cremegrundstoff die Migration bzw. Diffusion des Wirkstoffes in den Körper durch Wechselwirkung mit dem Wirkstoff verhindert oder erschwert, so daß hierdurch die Heilung verzögert oder erheblich verlängert wird, was bei der erfindungsgemäßen Zusammensetzung nicht auftritt. Hierin ist dann ein Grund zu sehen, warum die bekannten cremeartigen oder salbenartigen Zusammensetzungen im Vergleich zur erfindungsgemäßen Zusammensetzung allenfalls mit verringertem Erfolg zur Behandlung von lokal begrenzten Erkrankungen, die im Bereich der Haut lokalisiert sind, einsetzbar sind.

Im Vergleich zu den eingangs aufgeführten, systemisch wirkenden Darreichungsformen (z.B. Tablette, Dragee, Zäpfchen, Injektion, Infusion) weist die erfindungsgemäße Zusammensetzung den entscheidenden Vorteil auf, daß sie ohne die zuvor genannten Nebenwirkungen und Beeinträchtigungen über einen unbegrenzten Zeitraum anwendbar ist, da die erfindungsgemäße Zusammensetzung den Wirkstoff nicht über den Magen-/Darm-Trakt oder durch direktes Injizieren in die Blutbahn sondern durch Auftragen auf eine entsprechende Haut oder Schleimhaut dem Körper zuführt, so daß dann anschließend nach Transport des Wirkstoffes durch die Haut bzw. die Schleimhaut dieser Wirkstoff dann durch die Körperflüssigkeit, insbesondere das Blut, zum Wirkort transportiert wird und somit die erwünschte systemische Wirkung herbeiführt.

Eine erste Ausführungsform der erfindungsgemäßen pharmazeutischen Zusammensetzung sieht vor, daß hierbei die Zusammensetzung innerhalb einer Zeit von weniger als 2 Sekunden, vorzugsweise innerhalb einer Zeit zwischen 1 Sekunden und 0,01 Sekunden, auf der besprühten Körperfläche, insbesondere der besprühten Haut oder Schleimhaut, die Zubereitung ausbildet. Hierbei findet somit während der topischen Applikation oder unmittelbar danach innerhalb der zuvor genannten Zeiten das Verdampfen bzw. Verdunsten der in der flüssigen erfindungsgemäßen Zusammensetzung enthaltenen Flüssigkeit statt, so daß aufgrund der zuvor genannten relativ kurzen Zeiten selbst bei einer ungewollten Überdosierung oder der Anwendung an stark gewölbten oder geneigten Körperflächen verhindert wird, daß ein Teil der erfindungsgemäßen flüssigen Zusammensetzung von der jeweils besprühten Körperfläche abläuft oder unerwünscht verläuft.

Eine bevorzugte Weiterbildung der erfindungsgemäßen Zusammensetzung sieht vor, daß die flüssige erfindungsgemäße Zusammensetzung vor dem Versprühen eine Viskosität zwischen 1 mPas und 100 mPas, vorzugsweise zwischen 5 mPas und 25 mPas, aufweist.

Abhängig von dem jeweils mindestens einen Wirkstoff, der verwendeten Flüssigkeit bzw. des verwendeten Flüssigkeitsgemisches und den möglichen weiteren Inhaltsstoffen, so zum Beispiel dem noch später im Detail beschriebenen phospholipidischen Gelbildner, stellt die erfindungsgemäße flüssige Zusammensetzung eine Dispersion, Lösung und/oder eine Suspension dar. Hierbei enthält die erfindungsgemäße flüssige Zusammensetzung als Flüssigkeit bzw. als Flüssigkeitsgemisch ein pharmazeutisch unbedenkliches organisches Lösungsmittel bzw. ein pharmazeutisch unbedenkliches Lösungsmittelgemisch und/oder Wasser.

Vorzugsweise enthält die erfindungsgemäße flüssige Zusammensetzung als organisches Lösungsmittel bzw. als organisches Lösungsmittelgemisch einen oder mehrere Alkohole, der bzw. die leicht verdampfbar ist bzw. sind. Als besonders geeignet haben sich hierfür die leicht verdampfbaren, niedermolekularen Alkohole, wie insbesondere Ethanol, Propanol-1, Propanol-2 und/oder Butanole erwiesen, wobei vorzugsweise Ethanol oder Propanol-2 oder eine Mischung davon verwendet wird. Auch ist es möglich, als organisches Lösungsmittel in der erfindungsgemäßen Zusammensetzung Propylenglykol allein oder in Mischung mit den zuvor genannten Alkoholen vorzusehen. Sollte die erfindungsgemäße flüssige Zusammensetzung als organisches Lösungsmittel Butanole enthalten, so empfiehlt es sich, die Konzentration dieser Butanole in der erfindungsgemäßen Zusammensetzung auf einen oberen Grenzwert von etwa 15 Gew.%, bezogen auf die anwendungsfertige flüssige Zusammensetzung, zu begrenzen, um so die eingangs genannten Ausbildungszeiten der Zubereitung auf der besprühten Körperfläche innerhalb kürzester Zeit, vorzugsweise innerhalb von weniger als 4 Sekunden und insbesondere innerhalb einer Zeit von weniger als 2 Sekunden und vorzugsweise innerhalb einer Zeit zwischen 1 Sekunde und 0,01 Sekunden, einzuhalten.

Bezüglich der Gesamtkonzentration des Alkohols bzw. des Alkoholgemisches in der erfindungsgemäßen flüssigen Zusammensetzung ist festzuhalten, daß diese vorzugsweise zwischen 5 Gew.% und 40 Gew.% und insbesondere zwischen 7 Gew.% und 30 Gew.% variiert. Um bei besonders empfindlichen Patienten, bei denen Hautirritationen durch Alkohole ausgelöst werden können, das Auftreten dieser Hautirritationen zu vermeiden, kann der Gewichtsanteil des Alkohols bzw. des Alkoholgemisches noch weiter reduziert werden, so daß bei dieser besonders bevorzugten Ausführungsform der erfindungsgemäßen flüssigen Zusammensetzung die Konzentration des Alkohols bzw. Alkoholgemisches zwischen 13 Gew.% und 18 Gew.%, bezogen auf die anwendungsfertige flüssige Zusammensetzung, variiert.

Selbstverständlich können die zuvor genannten pharmazeutisch unbedenklichen organischen Lösungsmittel in der erfindungsgemäßen flüssigen Zusammensetzung auch durch Wasser ersetzt oder mit Wasser vermischt werden. Weist die erfindungsgemäße Zusammensetzung keinen Alkohol sondern lediglich Wasser auf, so ist hierbei die Gefahr des Auftretens von Hautirritationen bei besonders empfindlichen Patienten völlig eliminiert, so daß in diesem Fall dann die erfindungsgemäße Zusammensetzung neben dem Wasser und dem phospholipidischen Gelbildner lediglich nur noch den mindestens einen Wirkstoff enthält, sofern hierzu nicht zusätzlich noch die nachfolgend noch beschriebenen weiteren Innhaltsstoffe der erfindungsgemäßen Zusammensetzung zugesetzt werden.

Enthält jedoch die erfindungsgemäße Zusammensetzung ein Gemisch aus Alkoholen und Wasser, so variiert die Alkoholkonzentration in der erfindungsgemäßen Zusammensetzung unter Berücksichtigung der vorstehenden Ausführungen zwischen 5 Gew.% und 40 Gew.% und die Wasserkonzentration zwischen 90 Gew.% und 50 Gew.%, jeweils bezogen auf die anwendungsfertige flüssige Zusammensetzung, wobei die zu 100 Gew.% noch fehlenden Anteile dann auf den mindestens einen Wirkstoff und auf den noch in der erfindungsgemäßen Zusammensetzung enthaltenen phospholipidischen Gelbildner, wie dieser nachfolgend noch im Detail beschrieben ist, und auf weitere Inhaltsstoffe, wie insbesondere Konservierungsmittel oder Puffer, entfallen.

Bei den zuvor beschriebenen erfindungsgemäßen flüssigen Zusammensetzungen, bei denen nach dem nebelartigen Versprühen auf der besprühten Körperfläche gelartige Zubereitungen ausgebildet werden, variiert die Konzentration des mindestens einen phospholipidischen Gelbildners in der Zusammensetzung vorzugsweise zwischen 2 Gew.% und 14 Gew.%, jeweils bezogen auf die anwendungsfertige flüssige Zusammensetzung vor ihrem Versprühen. Mit anderen Worten enthalten somit bevorzugt derartige flüssige Zusammensetzungen
zwischen 0,5 Gew.% und 20 Gew.% des mindestens einen phospholipidischen Gelbildners,
zwischen 0 Gew.% und 40 Gew.% eines pharmazeutisch unbedenklichen organischen Lösungsmittels oder Lösungsmittelgemischs der vorstehend beschriebenen Art, den mindestens einen Wirkstoff der vorstehend genannten Art und der wier angegebenen Konzentrationen,
zwischen 0 Gew.% und 2 Gew.% eines Konservierungsmittels
   und/oder Puffers, sowie
Wasser in einer Gewichtsmenge, die zusammen mit den vorstehend genannten Bestandteilen 100 Gew.% ergibt.

Bezüglich des vorstehend genannten pharmazeutisch unbedenklichen organischen Lösungsmittels bzw. Lösungsmittelgemisches gelten auch für diese Weiterbildungen der erfindungsgemäßen flüssigen Zusammensetzung identisch die Ausführungen, wie sie vorstehend beschrieben sind.

Besonders geeignet ist es jedoch, wenn die erfindungsgemäße flüssige Zusammensetzung als phospholipidischen Gelbildner ein natürliches Phospholipid oder ein Phospholipidgemisch, und insbesondere ein aus Soja-Bohnen, Sonnenblumen und/oder Eiern isoliertes Phospholipid bzw. Phospholipidgemisch, enthält. Derartige phospholipidische Gelbildner weisen den Vorteil auf, daß sie einerseits eine hohe Haut- und Gewebeverträglichkeit besitzen, die Penetration des mindestens einen Wirkstoff in die Haut und/oder durch die Hautbarriere in die Körperflüssigkeit und/oder insbesondere ins Blut fördern und darüber hinaus noch den mindestens einen Wirkstoff wirkungsvoll gegen einen chemischen Abbau schützen. Desweiteren konnte überraschend festgestellt werden, daß durch Anwendung derartiger phospholipidischer Gelbildner ermöglicht wird, daß transparente flüssige Zusammensetzungen erstellt werden, die dementsprechend leicht auf Fremdpartikel kontrollierbar sind, so daß hierbei ein Verstopfen der zum Versprühen der erfindungsgemäßen flüssigen Zusammensetzung eingesetzten Düse einer entsprechenden Sprüheinrichtung verhindert wird. Auch neigen diese phospholipidischen Gelbildner nicht dazu, daß sie ihrerseits in der Sprühdüse austrocknen und somit die Austrittsdüse der Öffnung verschließen, was darauf zurückgeführt wird, daß die phospholipidischen Gelbildner, von denen nachfolgend noch besonders bevorzugte Ausführungsformen beschrieben sind, eine hohe Beständigkeit aufgrund ihrer Hydrophilie besitzen und zudem noch selbst dann, wenn die darin gebundene Flüssigkeit verdampft ist, wieder leicht beim erneuten Versprühen von der in der flüssigen Zusammensetzung enthaltenen Flüssigkeit aufgenommen werden können, so daß dementsprechend die Düse bei einem erneuten Versprühen sofort wieder von der ausgetrockneten Zusammensetzung befreit wird.

Bei einer besonders geeigneten Ausführungsform der zuvor beschriebenen Weiterbildung der erfindungsgemäßen Zusammensetzung weist die erfindungsgemäße flüssige Zusammensetzung als Gelbildner ein Phospholipid bzw. ein Phospholipidgemisch auf, das mindestens 60 Gew.% Phosphatidylcholin, vorzugsweise mindestens 76 Gew.% Phosphatidylcholin und insbesondere mindestens 90 Gew.% Phosphatidylcholin enthält, wobei sich diese Konzentrationsangaben des Phosphatidylcholins auf die Konzentration des phospholipidischen Gelbildners in der erfindungsgemäßen flüssigen Zusammensetzung beziehen.

Insbesondere dann, wenn bei einer anderen Ausführungsform der erfindungsgemäßen Zusammensetzung die Zusammensetzung ein Phospholipid bzw. ein Phospholipidgemisch als mindestens einen Gelbildner enthält, das mindestens aus 95 Gew.% Phosphatidylcholin besteht, werden die zuvor beschriebenen positiven Eigenschaften dieser Weiterbildung der erfindungsgemäßen Zusammensetzung noch weiter verbessert.

Um bei der erfindungsgemäßen flüssigen Zusammensetzung, die als Gelbildner die zuvor genannten Phospholipide mit den dort aufgeführten hohen Konzentration an Phosphatidylcholin aufweist, die eingangs beschriebenen Feinstverteilung des mindestens einen Wirkstoffes reproduzierbar sicherzustellen, sieht eine Variation der erfindungsgemäßen Zusammensetzung vor, daß hierbei die Konzentration an Lysophosphatidylcholin in dem phospholipidischen Gelbildner auf einen maximalen Wert von 6 Gew.%, insbesondere auf einen maximalen Wert von 4 Gew.%, bezogen auf die Menge des phospholipidischen Gelbildners in der erfindungsgemäßen flüssigen Zusammensetzung, begrenzt ist.

Phosphatidylcholin im Sinne der vorliegenden Beschreibung stellt chemisch gesehen 1,2-Diacylglycero-3-Phosphocholin (3-sn-Phosphatiylcholin) dar, wobei dieses 1,2-Diacylglycero-3-Phosphocholin entsprechende Acylreste sowohl in 1- als auch in 2-Stellung aufweisen kann.

Eine besonders bevorzugte Weiterbildung der erfindungsgemäßen flüssigen Zusammensetzung weist als phospholipidischen Gelbildner:ein Phosphatidylcholin auf, dessen Acylreste in 1-und 2-Stellung zu 10 bis 15 Gew.% aus dem Palmitinsäurerest, zu 1,5 bis 4 Gew.% aus dem Stearinsäurerest, zu 3 bis 10 Gew.% aus dem Ölsäurerest, zu 61 bis 71 Gew.% aus dem Linolsäurerest und zu 3 bis 7 Gew.% aus dem Linolensäurerest bestehen. Hierbei hängen die zuvor wiedergegebenen Schwankungen der Prozentangaben bei jedem Acylrest damit zusammen, daß das in der erfindungsgemäßen Zusammensetzung als phospholipidischer Gelbildner vorzugsweise eingesetzte Phosphatidylcholin ein solches Phosphatidylcholin ist, das aus natürlichen Quellen, insbesondere aus Soja-Bohnen, aus Sonnenblumen und/oder aus Eiern, isoliert ist und entsprechend gereinigt wurde.

Eine andere Ausführungsform der erfindungsgemäßen flüssigen Zusammensetzung beinhaltet als phospholipidischen Gelbildner ein hydriertes Phospholipid bzw. ein hydriertes Phospholipidgemisch in den zuvor beim Gelbildner allgemein genannten Konzentrationen.

Bevorzugt kann bei der erfindungsgemäßen flüssigen Zusammensetzung als phospholipidische Gelbildner auch ein solches hydriertes Phospholipidgemisch eingesetzt werden, dessen Phosphatidylcholingehalt innerhalb der vorstehend beim nicht hydrierten Phospholipidgemisch angegebenen Grenzen variiert, wobei das Phosphatidylcholin dann bei dem hydrierten Phospholipidgemisch in der 1- und 2-Stellung als Acylreste zu 85 Gew.% ± 3 Gew.% den Stearinsäurerest und zu 15 Gew.% ± 2 Gew.% den Palmitinsäurerest enthält.

Wie bereits vorstehend dargelegt ist, weist die erfindungsgemäßen flüssige Zusammensetzung vorzugsweise als Gelbildner ein solches Phospholipidgemisch auf, das als Hauptbestandteil Phosphatidylcholin in den vorstehend genannten Konzentrationsangaben beinhaltet. Neben diesem Phosphatidylcholin sind dann vorzugsweise maximal 6 Gew.% Lysophosphatidylcholin, 0 bis 12 Gew.% Phosphatidylethanolamin, 0 bis 8 Gew.% Phosphatidylinositol und/oder 0 bis 8 Gew.% Phosphatidsäure enthalten, wobei die zuvor wiedergegebenen Schwankungen der Konzentrationsangaben der weiteren phospholipidischen Bestandteile dadurch erklärlich werden, daß das als Gelbildner eingesetzte Phospholipidgemisch vorzugsweise aus einer natürlichen Quelle isoliert wurde. Desweiteren können noch geringe Anteile an Ölen und/oder Sterinen in diesem Phospholipidgemisch enthalten sein, wobei sich die zuvor wiedergegebenen Konzentrationsangaben auf das als Gelbildner vorhandene Phospholipidgemisch selbst und nicht auf die anwendungsfertige erfindungsgemäße flüssige Zusammensetzung beziehen.

Bezüglich des in der vorliegenden Beschreibung verwendeten Begriffes Wasser ist festzuhalten, daß dieser Begriff Wasser nicht nur Wasser selbst, d.h. somit destilliertes oder entionisiertes Wasser, sondern auch alle wäßrigen Systeme, so insbesondere Salz- oder Pufferlösungen, vorzugsweise Phosphatpuffer, abdeckt.

Eine besonders vorteilhafte Weiterbildung der zuvor beschriebenen Ausführungsformen der erfindungsgemäßen flüssigen Zusammensetzung, die bei einer topischen Applikation beim und/oder nach dem Aufsprühen auf dem Körperbereich eine gelartige Zubereitung ausbildet, sieht vor, daß die flüssige Zusammensetzung eine Dispersion ist, die Liposomen enthält, wobei diese vorzugsweise klare bis leicht opale flüssige Zusammensetzung dann als Inhaltsstoffe den mindestens einen Wirkstoff, mindestens einen der zuvor beschriebenen phospholipidischen Gelbildner sowie die vorstehend beschriebenen Flüssigkeiten bzw. Flüssigkeitsgemische und ggf. noch Konservierungsstoffe oder Puffer in den vorstehend genannten Konzentrationen beinhaltet. Diese Weiterbildung der erfindungsgemäßen flüssigen Zusammensetzung bildet dann auf dem besprühten Körperbereich innerhalb der vorstehend genannten Zeiten ein vesikuläres Liposomengel aus, wodurch einerseits verhindert wird, daß die aufgesprühte flüssige Dispersion von der Körperfläche abläuft und andererseits sichergestellt wird, daß nicht nur der mindestens eine Wirkstoff in einer hohen Konzentration und einer extremen Feinverteilung durch die Hautbarriere transportiert und dann an den Behandlungsort gelangt sondern gleichzeitig noch, daß auf der besprühten Körperfläche ein Depot des mindestens einen Wirkstoffs vorhanden ist.

Bezüglich der Konzentration des zuvor beschriebenen phospholipidischen Gelbildners ist festzuhalten, daß diese Konzentration, wie bereits vorstehend dargelegt ist, zwischen 0,5 Gew.% und 20 Gew.%, insbesondere zwischen 2 Gew.% und 14 Gew.%, variiert. Besonders gute Ergebnisse lassen sich dann erzielen, wenn der phospholipidische Gelbildner in der erfindungsgemäßen flüssigen Zusammensetzung in einer Konzentration zwischen 5 Gew.% und 14 Gew.% und insbesondere in einer Konzentration zwischen 8:Gew.% und 12 Gew.%, vorhanden ist, wobei diese Konzentrationsangaben davon abhängig sind, welche Viskosität in der flüssigen Zusammensetzung eingestellt werden soll, welche Tröpfchengröße beim Versprühen erzielt werden soll und welchen chemischen Aufbau der phospholipidische Gelbildner aufweist. Besonders gute Ergebnisse und Heileffekte lassen sich dann erzielen, wenn der phospholipidische Gelbildner in der erfindungsgemäßen flüssigen Zusammensetzung in einer Konzentration von etwa 10 Gew.%, bezogen auf die anwendungsfertige flüssige Zusammensetzung, vorhanden ist.

Desweiteren ist festzuhalten, daß die erfindungsgemäße flüssige Zusammensetzung vorzugsweise einen pH-Wert aufweist, der zwischen 5,0 und 8,0 variiert und insbesondere bei etwa 6,5 liegt. Hierbei kann dieser pH-Wert durch Zusatz eines geeigneten Puffers eingestellt werden.

Bezüglich der Konzentration des in der erfindungsgemäßen Zusammensetzung enthaltenen mindestens einen Wirkstoffs ist festzuhalten, daß sich diese Konzentration einerseits nach dem jeweiligen Wirkstoff, d.h. dem Penetrationsvermögen des Wirkstoffes durch die Haut- oder Gewebebarriere, der Löslichkeit des Wirkstoffes und/oder der erforderlichen Behandlungskonzentration am Wirkort, und andererseits nach der jeweils zu behandelnden Erkrankung, der Art der Behandlung, d.h. lokal oder systemisch, und der Häufigkeit der jeweiligen täglichen Anwendung richtet. Üblicherweise variiert die Wirkstoffkonzentration in der erfindungsgemäßen Zusammensetzung zwischen 0,01 Gew.% und 20 Gew.%, vorzugsweise in einer Konzentration zwischen 0,5 Gew.% und 10 Gew.%.

Generell ist zu dem in der erfindungsgemäßen Zusammensetzung enthaltenen mindestens einen Wirkstoff festzuhalten, daß hierfür grundsätzlich jeder Wirkstoff geeignet ist, der bei einer topischen Applikation in der Lage ist, die Haut- oder Zellwandbarrieren zu durchdringen und eine lokale Wirksamkeit zu entfalten und/oder anschließend von einer geeigneten Körperflüssigkeit, insbesondere Blut, aufgenommen zu werden, um so dann zum jeweiligen Wirkort transportiert zu werden.

Nachfolgend werden bevorzugte Wirkstoffe genannt, die in der erfindungsgemäßen Zusammensetzung enthalten sein können, wobei die noch aufgeführten Wirkstoffe dann abhängig von dem jeweils erwünschten Behandlungseffekt und der jeweiligen Erkrankung ausgewählt werden, ohne daß jedoch hierdurch eine Einschränkung der vorliegenden Erfindung erfolgt.

Wie bereits eingangs beim Hauptanspruch näher erläutert ist, kann die erfindungsgemäße Zusammensetzung als mindestens einen Wirkstoff mindestens einen analgetischen und/oder einen antirheumatischen Wirkstoff aufweisen, der aus der Gruppe, bestehend aus Acemetacin, Diclofenac und seinen Salzen, vorzugsweise Diclofenac-Natrium und/oder Diclofenac-Diethylamin, Etofenamat, Flufenaminsäure, Ibuprofen, Racematformen und/oder Enantiomere von Ibuprofen, vorzugsweise S(+)-Ibuprofen, Indometacin, Ketoprofen, Racematformen und/oder Enantiomere von Ketoprofen; Piroxicam, Salicylsäure und/oder Derivate davon, vorzugsweise Acetylsalicylsäure und/oder 2-Hydroxyethylsalicylsäure, ausgewählt ist. Besonders bevorzugt weist die erfindungsgemäße Zusammensetzung einen Wirkstoff, ausgewählt aus der Gruppe der Cyclooxygenase-2 selektiven nichtsteroidalen Antirheumatika/Analgetika auf.

Zusätzlich zu diesen Wirkstoffen oder anstelle dieser Wirkstoff kann die erfindungsgemäße Zusammensetzung auch als Wirkstoff mindestens einen Opiatrezeptor-Agonisten und/oder Opiatrezeptor-Antagonisten enthalten, der aus der Gruppe, bestehend aus Buprenorphin, Fentanyl, Pentazocin, Pethidin, Tilidin, Tramadol und/oder Naloxon, ausgewählt ist.

Zusätzlich oder anstelle der zuvor genannten Wirkstoffe kann die erfindungsgemäße Zusammensetzung auch als Wirkstoff mindestens einen die Blutgerinnung hemmenden Stoff und/oder einen die Thrombozytenaggregation hemmenden Stoff aufweisen, der aus der Gruppe, bestehend aus Heparin, niedermolekulares Heparin, Heparin-Salze, vorzugsweise Heparin-Natrium und/oder Heparin-Calcium, Heparinoide und/oder Acetylsalicylsäure und/oder deren Ester, ausgewählt ist.

Ebenso ist es möglich, daß die erfindungsgemäße Zusammensetzung als Wirkstoff Heparin, niedermolekulares Heparin, Heparin-Salze, vorzugsweise Heparin-Natrium und/oder Heparin-Calcium und/oder Heparinoide enthält, wobei dieser Wirkstoff bzw. diese Wirkstoffe dann bei einer systemischen Anwendung in der erfindungsgemäßen Zusammensetzung vorzugsweise in einer Konzentration:zwischen 250.000 I.E. und 2.500.000 I.E. pro 100 g Zusammensetzung enthalten ist bzw. sind. Hierbei bedeutet die Abkürzung I.E. Internationale Einheiten. Derartige hohe Heparin-Konzentrationen lassen sich mit herkömmlichen Salben oder Cremes nicht applizieren, so daß sich Patienten, die beispielsweise nach einer Operation zur Thromboembolie-Prophylaxe oder zur Prophylaxe von thromboembolischer Erkrankungen, zur Therapie von arteriellen und/oder venösen Thrombosen, bei Embolien, bei Herzinfarkten und instabiler Angina pectoris, zur Gerinnungshemmung während der Behandlung oder Operation mit extrakorpoaralem Kreislauf und zur Behandlung von Blutgerinnungsstörungen als Folge einer Umsatzsteigerung von Thrombozyten sowie plasmatischen Gerinnungsfaktoren behandelt werden müssen, bei Anwendung der herkömmlichen Injektionen täglich mehreren Injektionen aussetzen müssen.

Soll hingegen das Heparin, das niedermolekulare Heparin, das Heparin-Salz, insbesondere das Heparin-Natrium und/oder das Heparin-Calcium zur lokalen Applikation bei Sport- und Unfallverletzungen, Blutergüssen, varikösen Symptomenkomplexen, oberflächlichen Thrombosen, Thrombophlebitiden, entzündlichen Infiltraten, Narbenverhärtungen und/oder Hämorrhoiden eingesetzt werden, so liegt dieser Wirkstoff bzw. das entsprechende Wirkstoffgemisch in einer Konzentration von wenigstens 20.000 I.E., vorzugsweise jedoch in einer Konzentration zwischen 120.000 I.E. und 240.000 I.E., in der erfindungsgemäßen Zusammensetzung vor.

Darüber hinaus ist es möglich, daß die erfindungsgemäße Zusammensetzung mindestens einen kortikoiden Wirkstoff aufweist, der aus der Gruppe, bestehend aus Dexamethason, Betamethason, Triamcinolon und/oder Clobetasol, ausgewählt ist.

Enthält die erfindungsgemäße Zusammensetzung als Wirkstoff mindestens einen Histaminantagonisten, so wird dieser vorzugsweise aus der Gruppe, bestehend aus Bamipinlactat, Chlorphenoxamin-HCl, Clemastinhydrogenfumarat, Dimetidindenmaleat, Loratadin und/oder Pheniraminhydrogenmaleat, ausgewählt.

Auch kann in der erfindungsgemäßen Zusammensetzung als Wirkstoff mindestens ein antidiabetischer Wirkstoff, insbesondere ein Wirkstoff auf der Basis der Insuline, enthalten sein.

Ebenso geeignet ist es, wenn die erfindungsgemäße Zusammensetzung als Wirkstoff mindestens ein Chemotherapeutikum, vorzugsweise mindestens ein Antibiotikum, ausgewählt aus der Gruppe, bestehend aus Erythomycin, Tetracyclin HCl, Neomycinsulfat, Bacitracin, Chloramphenicol; und/oder ein Zytostatikum und/oder mindestens einen Metastasenhemmer, aufweist.

Bei den dermatologisch wirksamen Stoffen (Dermatika), die in der erfindungsgemäßen Zusammensetzung als Wirkstoffe enthalten sein können, sind insbesondere neben den zuvor schon aufgeführten Antibiotika, Antimykotika und/oder neben den vorstehend beschriebenen Kortikoiden noch desweiteren speziell Tretionin, Isoprenalinsulfat, Lidocain HCl, Dithranol, Harnstoff und/oder die bekannten Enzympräparate zur Behandlung von Hautdefekten zu erwähnen, während zu den Lokalanästhetika insbesondere Lidocain-HCL, Benzocain und/oder Tetracain zählen.

Desweiteren kann in der erfindungsgemäßen Zusammensetzung mindestens ein Wirkstoff gegen grippale Infekte und/oder Erkältungskrankheiten, vorzugsweise ein Antitussivum, ein Expektorans und/oder Sekretolytikum, enthalten sein, wobei bei den Neuropathie-Präparaten die α-Liponsäure bevorzugt zu nennen ist.

Ebenso kann die erfindungsgemäße Zusammensetzung als Wirkstoff Chinin und/oder Thalidomid enthalten.

Ferner ist es möglich, daß die erfindungsgemäße Zusammensetzung als Wirkstoff mindestens ein regulatorisches Peptid und/oder seine Hemmstoffe aufweist, das bzw. die aus der Gruppe, bestehend aus Hypophysenvorderlappenhormone und/oder ihrer Hemmstoffe, Hypophysenhinterlappenhormone und/oder ihrer Hemmstoffe, Hypothalamushormone und/oder ihre Hemmstoffe, ausgewählt ist bzw.sind.

Wird die erfindungsgemäße Zusammensetzung zur systemischen Behandlung von Viruserkrankungen oder viruell bedingter Erkrankungen eingesetzt, so enthält die Zusammensetzung als antiviralen Wirkstoff Aciclovir.

Ferner ist:es möglich, daß die erfindungsgemäße Zusammensetzung als Wirkstoff Prostaglandine, vorzugsweise PGE₁ sowie seine Ester, insbesondere den Ethylester, aufweist.

Bei dem zuvor genannten Heparin handelt es sich entweder um Standardheparin mit einem Molekulargewicht zwischen etwa 3.000 und 30.000, vorzugsweise mit einem mittleren Molekulargewicht zwischen 12.000 und 15.000, und/oder um niedermolekulares Heparin mit einem mittleren Molekulargewicht von etwa 4.000 bis 6.000.

Liegt die erfindungsgemäße flüssige Zusammensetzung als Liposomendispersion vor oder enthält die Zusammensetzung Liposome, so weist sie insbesondere Vesikel mit einem mittlere Durchmesser zwischen 70 nm und 500 nm, vorzugsweise mit einem mittleren Durchmesser zwischen 80 nm und 120 nm, auf, wobei diese Vesikel insbesondere ein- bis dreischalig sind.

Zur Herstellung der zuvor beschriebenen flüssigen Zusammensetzung, die einen phospholipidischen Gelbildner enthalten, werden zuerst die Phospholipide in einem Teil des Wassers gequollen und vorhydratisiert, damit sich in der wäßrigen Umgebung die lamellaren Phasen ausbilden können. Anschließend wird der mindestens eine Wirkstoff in Wasser aufgenommen und zugesetzt. Die so erhaltene Mischung wird unter Eintrag hoher Energie, insbesondere mittels eines Hochdruck- oder Spalthomogenisators, homogenisiert. Nach hinreichender Homogenisation wird die noch ggf. fehlende Restmenge an Wasser, ggf. an Puffer und/oder das pharmazeutisch unbedenkliche organische Lösungsmittel bzw. organische Lösungsmittelgemisch zugesetzt. Sollten die gewünschten und vorstehend anhand ihrer Durchmesser quantifizierten Liposomen erzeugt werden, so empfiehlt es sich, durch Variation der Homogenisationszeit und durch entsprechenden Energieeintrag diese gewünschte Liposomengröße herzustellen.

Wie bereits wiederholt ausgeführt ist, wird die erfindungsgemäße flüssige Zusammensetzung unter Ausbildung von nebelartigen Tröpfchen versprüht. Hierfür wird insbesondere eine Sprühvorrichtung verwendet, die vorzugsweise ohne Treibmittel arbeitet und die einen Pumpzerstäuber umfaßt, wobei dann über eine geeignete Zerstäuberdüse pro Sprühstoß 20 bis 300 µl der flüssigen Zusammensetzung in einer Tropfengröße zwischen 10 µm und 100µm abgegeben wird.

Eine besonders bevorzugte und spezielle Ausführungsform der zuvor beschriebenen erfindungsgemäßen flüssigen Zusammensetzung, die zur lokalen Behandlung von Sport- und Unfallverletzungen, Blutergüssen, varikösen Symptomenkomplexen, oberflächlichen Thrombosen, Thrombophlebitiden, entzündlichen Infiltraten, Narbenverhärtungen und/oder Hämorrhoiden bevorzugt anwendbar ist, stellt eine Liposomendispersion dar, und weist etwa 10 Gew.%, bezogen auf die anwendungsfertige flüssige Zusammensetzung, eines phospholipidischen Gelbildners der vorstehend genannten Art auf, wobei dieser phospholipidische Gelbildner insbesondere zwischen 73 Gew.% und 80 Gew.% Phosphatidylcholin, bezogen auf die Gesamtmasse Gelbildner, enthält. Desweiteren weist die Ausführungsform wahlweise 120.000 I.E. oder 240.000 I.E. Heparin pro 100 g flüssiger Zusammensetzung auf, wobei in dieser flüssigen Zusammensetzung etwa 16 Gew.% Ethanol enthalten sind. Der verbleibende Anteil der Zusammensetzung ist dann Wasser, während der pH-Wert dieser speziellen Ausführungsform der flüssigen Zusammensetzung bei etwa 6,5 liegt. Dieser pH-Wert wird über einen geeigneten Phosphatpuffer (KH₂PO₄/Na₂HPO₄) eingestellt.

Vorteilhafte Weiterbildungen der erfindungsgemäßen flüssigen Zusammensetzung sind in den Unteransprüchen angegeben.

Die erfindungsgemäße flüssige Zusammensetzung wird nachfolgend anhand von Ausführungsbeispielen näher erläutert.

Der in den Ausführungsbeispielen 1 bis 6 eingesetzte phospholipidische Gelbildner wies folgende Zusammensetzung auf:
76 ± 3 Gew.% Phosphatidylcholin
0 - 6 Gew.-% Lysophosphatidylcholin
0 - 8 Gew.% Phosphatidsäure
0 - 4 Gew.% Phosphatidylethanolamin
etwa 9 Gew.% andere, nicht näher analysierte übliche Begleitlipide.

Das in dem phospholipidischen Gelbildner enthaltene Phosphatidylcholin besaß in der 1- und 2-Stellung insgesamt die nachfolgend aufgeführten Acylreste:
10 - 15 Gew.% Palmitinsäurerest
1,5 - 4 Gew.% Stearinsäurerest
6 - 13 Gew.% Ölsäurerest
61 - 71 Gew.% Linolsäurerest
4 - 7 Gew.% Linolensäurerest.

### Ausführungsbeispiel 1

Die nach Ausführungsbeispiel 1 hergestellte pharmazeutische Zusammensetzung wies folgende Inhaltsstoffe auf:
10 g phospholipidischer Gelbildner
16 g Ethanol
1 g Acemetacin
0,5 g Phosphatpuffer (fest) und
destilliertes Wasser (etwa 72,5 g) ad 100 g pharmazeutischer Zusammensetzung.

Um die zuvor wiedergegebene pharmazeutische Zusammensetzung gemäß Ausführungsbeispiel herzustellen, wurden die 10 g des phospholipidischen Gelbildners in etwa 30 Vol.% der Gesamtmenge des Wasser quellen gelassen. Zu diesem gequollenen phospholipidischen Gelbildner wurde dann der in Ethanol gelöste Wirkstoff Acemetacin zugesetzt. Anschließend wurde die so entstandene Mischung mit Hilfe eines Hochdruckhomogenisators bearbeitet, wobei hierbei die Restmenge Wasser sowie der Puffer zugesetzt wurde. Während dieser Homogenisation wurde ständig die Vesikelgröße kontrolliert, wobei nach Erreichen einer mittleren Teilchengröße mit einem Durchmesser von etwa 100 nm die Homogenisation abgebrochen wurde. Der pH-Wert der transpa renten, leicht opalen Dispersion betrug 6,5.

### Ausführungsbeispiel 2

Wie in Ausführungsbeispiel 1 beschrieben, wurde eine zweite Ausführungsform der pharmazeutischen Zusammensetzung hergestellt, wobei diese zweite Ausführungsform sich von der zuvor beschriebenen ersten Ausführungsform dadurch unterschied, daß die Menge des phospholipidischen Gelbildners von 10 g auf 7 g reduziert wurde und daß das 1 g Acemetacin durch 2 g S(+)-Ibuprofen ersetzt wurde.

Der pH-Wert der Dispersion betrug 7 (eingestellt mit Natronlauge). Das Herstellungsverfahren für die pharmazeutische Zusammensetzung gemäß Ausführungsbeispiel 2 entsprach dem Herstellungsverfahren gemäß Ausführungsbeispiel 1.

### Ausführungsbeispiel 3

Eine pharmazeutische Zusammensetzung, die
2 g Diclofenac-Natrium
5 g phospholipidischer Gelbildner
15 g Propanol-2
7 g Propylenglykol
Salzsäure zur Einstellung eines pH-Wertes von 6,9 sowie Wasser ad 100 g Zusammensetzung
aufwies, wurde, wie vorstehend beschrieben, hergestellt. Hierbei wies diese klare und transparente Dispersion eine Teilchengröße von etwa 80 nm (Teilchendurchmesser) auf.

### Ausführungsbeispiel 4

Die nach Ausführungsbeispiel 4 hergestellte pharmazeutische Zusammensetzung wies folgende Inhaltsstoffe auf:
2,5 g phospholipidischer Gelbildner
16 g Ethanol
1.600.000 I.E. Heparin-Natrium (Gehalt: min. 150 I.E./mg)
0,5 g Phosphatpuffer (fest) und
destilliertes Wasser (etwa 66 g) ad 100 g pharmazeutischer Zusammensetzung.

Um die zuvor wiedergegebene pharmazeutische Zusammensetzung gemäß Ausführungsbeispiel 4 herzustellen, wurden die 2,5 g des phospholipidischen Gelbildners in etwa 1/10 der Gesamtmenge des Wasser quellen gelassen. Zu diesem gequollenen phospholipidischen Gelbildner wurde dann das in nahezu der Gesamtmenge des Restwassers gelöste Heparin-Natrium zugesetzt. Anschließend wurde die so entstandene Mischung mit Hilfe eines Hochdruckhomogenisators bearbeitet. Während dieser Homogenisation wurde ständig die Vesikelgröße kontrolliert, wobei nach Erreichen einer mittleren Teilchengröße mit einem Durchmesser von etwa 75 nm die zuvor aufgeführte Menge Ethanol zugesetzt wurde. Anschließend wurde durch Zusatz des Phosphatpuffers der pH-Wert auf 6,5 eingestellt.

### Ausführungsbeispiel 5

Die nach Ausführungsbeispiel 5 hergestellte pharmazeutische Zusammensetzung wies folgende Inhaltsstoffe auf:
10 g phospholipidischer Gelbildner
16 g Ethanol
120.000 I.E. Heparin-Natrium (Gehalt: min. 120 I.E./mg)
0,5 g Phosphatpuffer (fest) und
destilliertes Wasser (etwa 72,5 g) ad 100 g pharmazeutischer Zusammensetzung.

Um die zuvor wiedergegebene pharmazeutische Zusammensetzung gemäß Ausführungsbeispiel 5 herzustellen, wurden die 10 g des phospholipidischen Gelbildners in etwa 1/10 der Gesamtmenge des Wasser quellen gelassen. Zu diesem gequollenen phospholipidischen Gelbildner wurde dann das in nahezu der Gesamtmenge des Restwassers gelöste Heparin-Natrium zugesetzt. Anschließend wurde die so entstandene Mischung mit Hilfe eines Hochdruckhomogenisators bearbeitet. Während dieser Homogenisation wurde ständig die Vesikelgröße kontrolliert, wobei nach Erreichen einer mittleren Teilchengröße mit einem Durchmesser von etwa 75 nm die zuvor aufgeführte Menge Ethanol zugesetzt wurde. Anschließend wurde durch Zusatz des Phosphatpuffers der pH-Wert auf 6,5 eingestellt.

### Ausführungsbeispiel 6

Wie in Ausführungsbeispiel 5 beschrieben, wurde eine sechste Ausführungsform der pharmazeutischen Zusammensetzung hergestellt, wobei diese sechst Ausführungsform sich von der zuvor beschriebenen fünften Ausführungsform dadurch unterschied, daß die Konzentration des Heparin-Natrium-Wirkstoffes bei 240.000 I.E. lag. Dementsprechend wies die sechste Ausführungsform eine minimal geringere Wassermenge auf.

Während die zuvor beschriebenen pharmazeutischen Zusammensetzungen gemäß der Ausführungsbeispiele 1 bis 4 überwiegend systemisch angewendet werden, beschreiben die Ausführungsbeispiele 5 und 6 solche pharmazeutischen Zusammensetzungen, die auf lokale Erkrankungen und/oder Verletzungen, insbesondere bei Sport- und Unfallverletzungen, Blutergüssen, varikösen Symptomenkomplexen, oberflächlichen Thrombosen, Thrombophlebitiden, entzündlichen Infiltraten, Narbenverhärtungen und/oder Hämorrhoiden, appliziert werden.

Alle sechs Ausführungsformen der nach Ausführungsbeispielen 1 bis 6 hergestellten Liposomendispersionen stellten klare bis leicht opale Dispersionen dar, die in einem herkömmlich ausgebildeten Pumpzerstäuber unter Ausbildung von nebelartigen Tröpfchen versprühbar waren. Pro Sprühstoß versprühte der Pumpzerstäuber etwa 200 µl der Dispersion.

Mit dem zuvor beschriebenen konventionellen Pumpzerstäuber wurde im Abstand von 30 cm eine senkrecht aufgestellte Glasplatte angesprüht, wobei zwischen dem Pumpzerstäuber und der Glasplatte exakt in der Mitte, d.h. somit 15 cm Abstand vom Pumpzerstäuber, ein Sieb angeordnet war, dessen Mesh-Weite 200 betrug.

Zunächst wurde der Pumpzerstäuber mit destilliertem Wasser gefüllt. Beim Besprühen der Glasplatte stellte sich jedoch hierbei heraus, daß der Sprühstoß das zuvor genannte Sieb nicht passierte und somit das Wasser an der senkrecht aufgestellten Siebfläche herunterlief.

Hiernach wurde der Pumpzerstäuber mit einer Lösung von Heparin-Natrium (800.000 I.E. pro 100 g Zusammensetzung) gefüllt und der vorstehend genannte Sprühversuch mehrfach wiederholt. Hierbei konnte festgestellt werden, daß etwa 50 Vol.% der pro Sprühstoß aufgesprühten Menge die Siebfläche passierte und auf die Glasplatte gelangte und dort so schnell eintrocknete, daß eine dünne, relativ gleichmäßige Schicht des Heparin-Natriums auf der Glasfläche ausgebildet wurde.

Der Pumpzerstäuber wurde mit der in Ausführungsbeispiel 1 beschriebenen Zusammensetzung gefüllt. Hiernach wurde die zuvor beschriebene Glasplatte wiederholt besprüht, wobei nur etwa 10 Vol.% der aufgesprühten Zusammensetzung gemäß Ausführungsbeispiel 1 von der Siebfläche zurückgehalten wurde. 90 Vol.% bildeten auf der Glasfläche spontan ein Gel aus, das nicht an der Glasfläche herablief.

Der Pumpzerstäuber wurde mit der in Ausführungsbeispiel 2 beschriebenen Zusammensetzung gefüllt. Beim Sprühversuch konnte festgestellt werden, daß hiervon lediglich etwa 15 Vol.% der pro Pumpstoß zerstäubten Menge die Siebfläche nicht passierte, während 85 Vol.% der aufgesprühten Menge auf die Glasplatte auftraf und dort spontan ein Gel ausbildete, das nicht von der Glasplatte ablief.

Der Pumpzerstäuber wurde mit der in Ausführungsbeispiel 3 beschriebenen Zusammensetzung gefüllt. Beim Sprühversuch konnte festgestellt werden, daß hiervon lediglich etwa 8 Vol.% der pro Pumpstoß zerstäubten Menge die Siebfläche nicht passierte, während 92 Vol.% der aufgesprühten Menge auf die Glasplatte auftraf und dort spontan ein Gel ausbildete, das nicht von der Glasplatte ablief.

Der Pumpzerstäuber wurde mit der in Ausführungsbeispiel 4 beschriebenen Zusammensetzung gefüllt. Beim Sprühversuch konnte festgestellt werden, daß hiervon lediglich etwa 6 Vol.% der pro Pumpstoß zerstäubten Menge die Siebfläche nicht passierte, während 94 Vol.% der aufgesprühten Menge auf die Glasplatte auftraf und dort spontan ein Gel ausbildete, das nicht von der Glasplatte ablief.

Der Pumpzerstäuber wurde mit der in Ausführungsbeispiel 5 beschriebenen Zusammensetzung gefüllt. Beim Sprühversuch konnte festgestellt werden, daß hiervon lediglich etwa 10 Vol.% der pro Pumpstoß zerstäubten Menge die Siebfläche nicht passierte, während 90 Vol.% der aufgesprühten Menge auf die Glasplatte auftraf und dort spontan ein Gel ausbildete, das nicht von der Glasplatte ablief.

Der Pumpzerstäuber wurde mit der in Ausführungsbeispiel 6 beschriebenen Zusammensetzung gefüllt. Beim Sprühversuch konnte festgestellt werden, daß hiervon lediglich etwa 5 Vol.% der pro Pumpstoß zerstäubten Menge die Siebfläche nicht passierte, während 95 Vol.% der aufgesprühten Menge auf die Glasplatte auftraf und dort spontan ein Gel ausbildete, das nicht von der Glasplatte ablief. ,

Aus den vorstehend wiedergegebenen Ergebnissen ist zu schließen, daß nur das mit dem eingesetzten Pumpzerstäuber zerstäubte Wasser beim Versprühen Tröpfchen ergab, dessen Tröpfchendurchmesser über etwa 74 µm lagen. Sowohl die wäßrige Heparinlösung als auch die nach den Ausführungsbeispielen 1 bis 6 hergestellten Zusammensetzungen wiesen somit Tröpfchen auf, deren Durchmesser kleiner als 74 µm waren.

In einem Probandenversuch, bestehend aus 10 weiblichen Probanden, die alle an einer chronischen Venenentzündung litten, wurde die in Ausführungsbeispiel 5 beschriebene Zusammensetzung erprobt. Hierbei wurde die Zusammensetzung gemäß Ausführungsbeispiel 5 in den zuvor genannten Pumpzerstäuber abgefüllt, wobei die Probandinnen gebeten wurden, täglich dreimal jeweils drei Pumpstöße aufzutragen, so daß pro Tag insgesamt etwa 1.800 µl der Zusammensetzung aufgesprüht wurde.

Alle Probandinnen hatten bisher ihre Venenentzündung mit einer herkömmlichen Heparin-haltigen Salbe mit einem Heparin-Gehalt von 120.000 I.E. behandelt.

Schon bereits zu Beginn der Erprobung berichteten 8 Probandinnen übereinstimmend und unabhängig voneinander, daß sie die Anwendung der Sprüh-Dispersion wesentlich angenehmer und weniger zeitaufwendig empfanden als die bisher verwendete Heparin-haltige Salbe. Insbesondere stellten die Probandinnen fest, daß es zu keinem unerwünschten Verschmieren von Kleidungsstücken kam, so daß die Sprühdispersion auch während der Arbeitszeit angewandt wurde. Desweiteren berichteten alle Probandinnen übereinstimmend, daß nach Aufsprühen der Zusammensetzung gemäß Ausführungsbeispiel 5 eine Kühlung und sofortige Schmerzlinderung auftrat, was bei Verwendung der herkömmlichen Salbe nicht der Fall war. Sechs Probandinnen, die zuvor über einen Zeitraum von vier Wochen die Heparin-haltige Salbe verwandt hatten und die bis zur Anwendung der Sprühdispersion nur eine geringfügige Heilung feststellten, waren nach Anwendung der Sprühdispersion gemäß Ausführungsbeispiel 5 innerhalb von zwölf Tagen geheilt.

## Patentansprüche

1. Pharmazeutische Zusammensetzung mit mindestens einem systemisch und/oder lokal wirkenden, topisch applizierbaren Wirkstoff, vorzugsweise ausgewählt aus der Gruppe, bestehend aus den Lokalanästhetika, Antiallergika, Dermatika, Wirkstoffen gegen grippale Infekte und Erkältungskrankheiten, Wirkstoffen zur Behandlung von Neuropathien, Chemotherapeutika, Chinin, Thalidomid, Analgetika, nichtsteroidalen Antirheumatika, Opiatrezeptor-Agonisten, Opiatrezeptor-Antagonisten, Blutgerinnung hemmenden Stoffen, Thrombozytenaggregation hemmenden Stoffen, Kortikoiden, Histaminantagonisten, Antidiabetika, regulatorischen Peptiden und ihren Hemmstoffen, Prostaglandinen und/oder ihren Estern und/oder antiviral wirksamen Stoffen, wobei die Zusammensetzung eine solche flüssige Konsistenz aufweist, daß sie als Tröpfchen auf eine Körperfläche versprühbar ist, **dadurch gekennzeichnet, daß** die flüssige Zusammensetzung einen Gelbildner auf der Basis eines Phospholipids oder eines Phospholipidgemisches in einer Konzentration zwischen 0,5 Gew.% und 20 Gew.% aufweist, daß die Zusammensetzung als Tröpfchen mit einem Tröpfchendurchmesser zwischen 10 µm und 100µm versprühbar ist und daß die Zusammensetzung nach dem Versprühen innerhalb kürzester Zeit, vorzugsweise innerhalb einer Zeit von weniger als 4 Sekunden, auf der besprühten Körperfläche, insbesondere auf der besprühten Haut oder Schleimhaut, eine solche Zubereitung ausbildet, in der der Wirkstoff dann in einer Feinverteilung vorliegt und in der die Konzentration des mindestens einen Wirkstoffes zwischen 25 % und 150 % höher ist als die Konzentration des mindestens einen Wirkstoffs in der ursprünglichen flüssigen Zusammensetzung vor dem Versprühen.

2. Pharmazeutische Zusammensetzung nach Anspruch 1, **dadurch gekennzeichnet, daß** die Zusammensetzung innerhalb einer Zeit von weniger als 2 Sekunden, vorzugsweise innerhalb einer Zeit zwischen 1 Sekunden und 0,01 Sekunden, auf der besprühten Körperfläche die Zubereitung ausbildet.

3. Pharmazeutische Zusammensetzung nach Anspruch 1 oder 2, **dadurch gekennzeichnet, daß** die Zusammensetzung vor dem Versprühen eine Viskosität zwischen 1 mPas und 100 mPas, vorzugsweise zwischen 5 mPas und 25 mPas, aufweist.

4. Pharmazeutische Zusammensetzung nach einem der vorangehenden Ansprüche, **dadurch gekennzeichnet, daß** die Zusammensetzung neben dem mindestens einen Wirkstoff und dem phospholipidischen Gelbildner lediglich mindestens eine Flüssigkeit oder ein Flüssigkeitsgemisch enthält, so daß die auf der besprühten Körperfläche ausgebildete Zubereitung aus dem mindestens einen Wirkstoff besteht.

5. Pharmazeutische Zusammensetzung nach einem der vorangehenden Ansprüche, **dadurch gekennzeichnet, daß** die flüssige Zusammensetzung eine Dispersion, Lösung und/oder eine Suspension ist und daß die Zusammensetzung als Flüssigkeit bzw. Flüssigkeitsgemisch ein pharmazeutisch unbedenkliches organisches Lösungsmittel bzw. Lösungsmittelgemisch und/oder Wasser enthält.

6. Pharmazeutische Zusammensetzung nach Anspruch 4 oder 5, **dadurch gekennzeichnet, daß** das organische Lösungsmittel einen Alkohol oder ein Alkoholgemisch umfaßt, der bzw. das leicht verdampfbar ist.

7. Pharmazeutische Zusammensetzung nach einem der Ansprüche 4 bis 6, **dadurch gekennzeichnet, daß** die Konzentration des Alkohols bzw. des Alkoholgemisches in der flüssigen Zusammensetzung zwischen 5 Gew.% und 40 Gew.%, bezogen auf die flüssige Zusammensetzung, variiert.

8. Pharmazeutische Zusammensetzung nach einem der Ansprüche 4 bis 7, **dadurch gekennzeichnet, daß** die flüssige Zusammensetzung zwischen 90 Gew.% und 50 Gew.% Wasser enthält.

9. Pharmazeutische Zusammensetzung nach einem der vorangehenden Anspruche, **dadurch gekennzeichnet, daß** die Konzentration des phospholipidischen Gelbildners in der Zusammensetzung zwischen 2 Gew.% und 14 Gew.%, bezogen auf die flüssige Zusammensetzung, variiert.

10. Pharmazeutische Zusammensetzung nach einem der vorangehenden Ansprüche, **dadurch gekennzeichnet, daß** das in der Zusammensetzung als Gelbildner vorhandene Phospholipid oder Phospholipidgemisch ein aus Soja-Bohnen, Sonnenblumen und/oder Ei isoliertes Phospholipid bzw. Phospholipidgemisch ist.

11. Pharmazeutische Zusammensetzung nach einem der vorangehenden Ansprüche, **dadurch gekennzeichnet, daß** die Zusammensetzung als Gelbildner ein Phospholipid bzw. ein Phospholipidgemisch aufweist, das mindestens 60 Gew.% Phosphatidylcholin, vorzugsweise mindestens 76 Gew.% Phosphatidylcholin und insbesondere mindestens 90 Gew.% Phosphatidylcholin, enthält.

12. Pharmazeutische Zusammensetzung nach Anspruch 11, **dadurch gekennzeichnet, daß** die Zusammensetzung als Gelbildner ein Phospholipid bzw. ein Phospholipidgemisch aufweist, das mindestens 95 Gew.% Phosphatidylcholin enthält.

13. Pharmazeutische Zusammensetzung nach einem der vorangehenden Ansprüche **dadurch gekennzeichnet, daß** das Phospholipidgemisch maximal 6 Gew.% Lysophosphatidylcholin, vorzugsweise maximal 4 Gew.% Lysophosphatidylcholin, aufweist.

14. Pharmazeutische Zusammensetzung nach einem der vorangehenden Ansprüche 11 bis 13, **dadurch gekennzeichnet, daß** die Zusammensetzung ein Phosphatidylcholin aufweist, dessen Acylreste in 1- und 2-Stellung zu 10 - 15 Gew.% aus dem Palmitinsäurerest, zu 1,5 - 4 Gew.% aus dem Stearinsäurerest, zu 3 - 10 Gew.% aus dem Ölsäurerest, zu 61 - 71 Gew.% aus dem Linolsäurerest und zu 3 - 7 Gew.% aus dem Linolensäurerest bestehen.

15. Pharmazeutische Zusammensetzung nach einem der vorangehenden Ansprüche **dadurch gekennzeichnet, daß** die Zusammensetzung als Gelbildner ein hydriertes Phospholipid bzw. Phospholipidgemisch aufweist.

16. Pharmazeutische Zusammensetzung nach Anspruch 15, **dadurch gekennzeichnet, daß** das in dem hydrierten Phospholipid bzw. Phospholipidgemisch enthaltene Phosphatidylcholin in der 1-und in der:2-Stellung als Acylreste zu 85 Gew.% ± 3 Gew.% den Stearinsäurerest und zu 15 Gew.% ± 2 Gew.% den Palmitinsäurerest enthält.

17. Pharmazeutische Zusammensetzung nach einem der Ansprüche 13 bis 19, **dadurch gekennzeichnet, daß** das Phospholipidgemisch neben Phosphatidylcholin als Hauptbestandteil und den maximal 6 Gew.% Lysophosphatidylcholin desweiteren 0 - 12 Gew.% Phosphatidylethanolamin, 0 - 8 Gew.% Phosphatidylinositol und/oder 0 - 8 Gew.% Phosphatidsäure aufweist.

18. Pharmazeutische Zusammensetzung nach einem der vorangehenden Ansprüche **dadurch gekennzeichnet, daß** die flüssige Zusammensetzung eine Dispersion ist und Liposomen enthält.

19. Pharmazeutische Zusammensetzung nach einem der vorangehenden Ansprüche, **dadurch gekennzeichnet, daß** die Zusammensetzung den Wirkstoff in einer Konzentration zwischen 0,01 Gew.% und 20 Gew.%, vorzugsweise in einer Konzentration zwischen 0,5 Gew.% und 10 Gew.%, enthält.

20. Pharmazeutische Zusammensetzung nach einem der vorangehenden Ansprüche, **dadurch gekennzeichnet, daß** die Zusammensetzung mindestens einen analgetischen und/oder antirheumatischen Wirkstoff aufweist, der aus der Gruppe, bestehend aus Acemetacin, Diclofenac und seinen Salzen, vorzugsweise Diclofenac-Natrium und/oder Diclofenac-Diethylamin, Etofenamat, Flufenaminsäure, Ibuprofen, Racematformen und/oder Enantiomere von Ibuprofen, vorzugsweise S(+)-Ibuprofen, Indometacin, Ketoprofen, Racematformen und/oder Enantiomere von Ketoprofen, Piroxicam, Salicylsäure und/oder Derivate davon, vorzugsweise Acetylsalicylsäure und/oder 2-Hydroxyethylsalicylsäure, und/oder der Cyclooxygenase-2 selektiven nichtsteroidalen Antirheumatika/Antiallergika, ausgewählt ist.

21. Pharmazeutische Zusammensetzung nach einem der vorangehenden Ansprüche, **dadurch gekennzeichnet, daß** die Zusammensetzung als Wirkstoff mindestens einen Opiatrezeptor-Agonisten und/oder Opiatrezeptor-Antagonisten aufweist, der aus der Gruppe, bestehend aus Buprenorphin, Fentanyl, Pentazocin, Pethidin, Tilidin, Tramadol und/oder Naloxon, ausgewählt ist.

22. Pharmazeutische Zusammensetzung nach einem der vorangehenden Ansprüche, **dadurch gekennzeichnet, daß** die Zusammensetzung als Wirkstoff mindestens einen die Blutgerinnung hemmenden Stoff und/oder einen die Thrombozytenaggregation hemmenden Stoff aufweist, der aus der Gruppe, bestehend aus Heparin, niedermolekulares Heparin, Heparin-Salze, vorzugsweise Heparin-Natrium und/oder Heparin-Calcium, Heparinoide und/oder Acetylsalicylsäure und/oder deren Ester, ausgewählt ist.

23. Pharmazeutische Zusammensetzung nach Anspruch 22, **dadurch gekennzeichnet, daß** in der Zusammensetzung das Heparin, niedermolekulares Heparin, Heparin-Salze, vorzugsweise Heparin-Natrium und/oder Heparin-Calcium und/oder die Heparinoide in einer Konzentration zwischen 250.000 I.E. und 2.500.000 I.E. pro 100 g Zusammensetzung enthalten ist.

24. Pharmazeutische Zusammensetzung nach einem der Ansprüche 1 bis 22, **dadurch gekennzeichnet, daß** die Zusammensetzung zur lokalen Applikation bei Sport- und Unfallverletzungen, Blutergüssen, varikösen Symptomenkomplexen, oberflächlichen Thrombosen, Thrombophlebitiden, entzündlichen Infiltraten, Narbenverhärtungen und/oder Hämorrhoiden das Heparin, das niedermolekulares Heparin, die Heparin-Salze, vorzugsweise das Heparin-Natrium und/oder das Heparin-Calcium, und/oder die Heparinoide in einer Konzentration von wenigstens 20.000 I.E. enthält.

25. Pharmazeutische Zusammensetzung nach Anspruch 23, **dadurch gekennzeichnet, daß** die Zusammensetzung das Heparin, das niedermolekulares Heparin, die Heparin-Salze, vorzugsweise das Heparin-Natrium und/oder das Heparin-Calcium, und/oder die Heparinoide in einer Konzentration zwischen 120.000 I.E. und 240.000 I.E. enthält.

26. Pharmazeutische Zusammensetzung nach einem der vorangehenden Ansprüche, **dadurch gekennzeichnet, daß** die Zusammensetzung mindestens einen kortikoiden Wirkstoff aufweist, der aus der Gruppe, bestehend aus Dexamethason, Betamethason, Triamcinolon und/oder Clobetasol, ausgewählt ist.

27. Pharmazeutische Zusammensetzung nach einem der vorangehenden Ansprüche, **dadurch gekennzeichnet, daß** die Zusammensetzung als Wirkstoff mindestens einen Histaminantagonisten aufweist, der aus der Gruppe, bestehend aus Bamipinlactat, Chlorphenoxamin-HCl, Clemastinhydrogenfumarat, Dimetidindenmaleat, Loratadin und/oder Pheniraminhydrogenmaleat, ausgewählt ist.

28. Pharmazeutische Zusammensetzung nach einem der vorangehenden Ansprüche, **dadurch gekennzeichnet, daß** die Zusammensetzung als Wirkstoff mindestens einen antidiabetischen Wirkstoff, vorzugsweise ein Insulin, enthält.

29. Pharmazeutische Zusammensetzung nach einem der vorangehenden Ansprüche, **dadurch gekennzeichnet, daß** die Zusammensetzung als Wirkstoff mindestens einen dermatologisch wirksamen Stoff enthält.

30. Pharmazeutische Zusammensetzung nach einem der vorangehenden Ansprüche, **dadurch gekennzeichnet, daß** die Zusammensetzung mindestens ein Lokalanästhetikum, vorzugsweise Lidocain-HCL, Benzocain und/oder Tetracain, enthält.

31. Pharmazeutische Zusammensetzung nach einem der vorangehenden Ansprüche, **dadurch gekennzeichnet, daß** die Zusammensetzung als Wirkstoff Chinin und/oder Thalidomid aufweist.

32. Pharmazeutische Zusammensetzung nach einem der vorangehenden Ansprüche, **dadurch gekennzeichnet, daß** die Zusammensetzung als Wirkstoff mindestens ein Chemotherapeutikum, vorzugsweise ein Antibiotikum, ein Zytostatika und/oder mindestens einen Metastasenhemmer enthält.

33. Pharmazeutische Zusammensetzung nach einem der vorangehenden Ansprüche, **dadurch gekennzeichnet, daß** die Zusammensetzung als Wirkstoff mindestens ein regulatorisches Peptid und/oder seine Hemmstoffe aufweist, das bzw. die aus der Gruppe, bestehend aus Hypophysenvorderlappenhormone und/oder ihrer Hemmstoffe, Hypophysenhinterlappenhormone und/oder ihrer Hemmstoffe, Hypothalamushormone und/oder ihre Hemmstoffe, ausgewählt ist.

34. Pharmazeutische Zusammensetzung nach einem der, vorangehenden Ansprüche, **dadurch gekennzeichnet, daß** die Zusammensetzung als antiviralen Wirkstoff Aciclovir aufweist.

35. Pharmazeutische Zusammensetzung nach einem der vorangehenden Ansprüche, **dadurch gekennzeichnet, daß** die Zusammensetzung als Wirkstoff Prostaglandine, vorzugsweise PGE₁ sowie seine Ester, insbesondere den Ethylester, aufweist.

## Claims

1. A pharmaceutical composition containing at least one systemically and/or locally effective, topically applicable active substance, preferably selected from the group consisting of local anaesthetics, anti-allergic agents, dermatics, active substances for influenzal infections and colds, active substance for the treatment of neuropathies, chemotherapeutics, quinine, thalidomide, analgesics, non-steroid antirheumatics, opiate receptor agonists, opiate receptor antagonists, substances inhibiting the blood coagulation, substances inhibiting platelet aggregation, corticoids, histamine antagonists, anti-diabetics, regulatory peptides and the inhibitors thereof, prostaglandins and/or the esters thereof and/or antivirally effective substances, whereby the composition has such a liquid consistency that it is sprayable in droplets on a body surface, **characterised in that** the liquid composition contains a gel-forming agent on the basis of a phospholipide or a phospholipide mixture in a concentration between 0,5 % by weight and 20 % by weight, that the composition is sprayable as a droplet with a droplet diameter of between 10 µm and 100 µm and that the composition forms a preparation within short time, preferably within a time of less than 4 seconds, on the sprayed body surface, particularly on the sprayed skin or mucous membrane, whereby, compared to the original liquid composition, this preparation is formed in such a preparation which contains the active substance in a finely divided way and in which the concentration of the at least one active substance is between 25 % and 150 % higher than the concentration of the at least active substance in the original liquid composition before the spraying.

2. The pharmaceutical composition according to claim 1, **characterised in that** the composition forms the preparation on the sprayed body surface within a time of less than 2 seconds, preferably within a time of between 1 second and 0,01 seconds.

3. The pharmaceutical composition according to claim 1 or 2, **characterised in that** before being sprayed the composition has a viscosity of between 1 mPas and 100 mPas, preferably between 5 mPas and 25 mPas.

4. The pharmaceutical composition according to one of the preceding claims, **characterised in that** the composition comprises only at least one liquid or a liquid mixture additionally to the at least one active substance and the at least phospholipidic gel-forming agent, so that the preparation formed on the sprayed body surface consists of at least one active substance.

5. The pharmaceutical composition according to one of the preceding claims, **characterised in that** the liquid composition is a dispersion, solution and/or a suspension and that the composition contains as liquid, respectively as liquid mixture, a pharmaceutically harmless organic solvent, respectively a solvent mixture, and/or water.

6. The pharmaceutical composition according to claim 4 or 5, **characterised in that** the organic solvent comprises an alcohol or an alcohol mixture which are easily vaporisable.

7. The pharmaceutical composition according to one of the claims 4 to 6, **characterised in that** the concentration of the alcohol, respectively the alcohol mixture, in the liquid composition varies between 5 % by weight and 40 % by weight relative to the liquid composition.

8. The pharmaceutical composition according to one of the claims 4 to 7, **characterised in that** the liquid composition contains between 90 % by weight and 50 % by weight water.

9. The pharmaceutical composition according to one of the preceding claims, **characterised in that** the concentration of the phospholipidic gel-forming agent in the composition varies between 2 % by weight and 14 % by weight relative to the liquid composition.

10. The pharmaceutical composition according to one of the preceding claims, **characterised in that** the phospholipide or the phospholipide mixture which is contained in the composition as gel-forming agent is such a phospholipide or a phospholipide mixture isolated from soybeans, sunflowers and/or from egg.

11. The pharmaceutical composition according to one of the preceding claims, **characterised in that** the composition comprises as gel-forming agent a phospholipide, respectively a phospholipide mixture, which contains at least 60 % by weight phosphatidylcholine, preferably at least 76 % by weight phosphatidylcholine and particularly at least 90 % by weight phosphatidylcholine.

12. The pharmaceutical composition according to claim 11, **characterised in that** the composition comprises as gel-forming agent a phospholipide, respectively a phospholipide mixture, which contains at least 95 % by weight phosphatidylcholine.

13. The pharmaceutical composition according to one of the preceding claims, **characterised in that** the phospholipide mixture comprises as a maximum 6 % by weight lysophosphatidylcholine, preferably as a maximum 4 % by weight lysophosphatidylcholine.

14. The pharmaceutical composition according to one of the claims 11 to 13, **characterised in that** the composition comprises a phosphatidylcholine which contains acyl residues in the 1- and 2-position that consist between 10 and 15 % by weight of the palimitic acid residue, between 1,5 and 4 % by weight of the stearic acid residue, between 3 and 10 % by weight of the oleic acid residue, between 61 and 71 % by weight of the linoleic acid residue and between 3 and 7 % by weight of the linolenic acid residue.

15. The pharmaceutical composition according to one of the preceding claims, **characterised in that** the composition comprises as gel-forming agent a hydrated phospholipide, respectively phospholipide mixture.

16. The pharmaceutical composition according to claim 15, **characterised in that** the phosphatidylcholine existing in the hydrated phospholipide, respectively phospholipide mixture, consists in the 1- and 2-position of 85 % by weight ± 3 % by weight of the stearic acid residue and of 15 % by weight ± 2 % by weight of the palmitic acid residue.

17. The pharmaceutical composition according to one of the claims 13 to 19, **characterised in that**, additionally to the phosphatidylcholine as main component and the at maximum 6 % by weight lysophosphatidylcholine, the phospholipide mixture comprises furthermore 0 - 12 % by weight phosphatidylethanolamine, 0 - 8 % by weight phosphatidylinositol and/or 0 - 8 % by weight phosphatidic acid.

18. The pharmaceutical composition according to one of the preceding claims, **characterised in that** the liquid composition is a dispersion and contains liposomes.

19. The pharmaceutical composition according to one of the preceding claims, **characterised in that** the composition comprises the active substance in a concentration of between 0,01 % by weight and 20 % by weight, preferably in a concentration of between 0,5 % by weight and 10 % by weight.

20. The pharmaceutical composition according to one of the preceding claims, **characterised in that** the composition comprises at least one analgesic and/or anti-rheumatic active substance which is selected from the group consisting of acemetacine, diclofenac and the salts thereof, preferably diclofenac-sodium and/or dicolfenac-diethylamine, etofenamate, flufenamic acid, ibuprofene, racemic forms and/or enantiomers of ibuprofene, preferably S(+)-ibuprofene, indometacine, ketoprofene, racemic forms and/or enantiomers of ketoprofene, piroxicam, salicylic acid and/or derivatives thereof, preferably acetylsalicylic acid and/or 2-hydroxyethylsalicylic acid, and/or from the cylcooxygenase-2 selective non-steroid anti-rheumatics/anti-allergics.

21. The pharmaceutical composition according to one of the preceding claims, **characterised in that** the composition comprises as active substance at least one opiate receptor agonist and/or opiate receptor antagonist which is selected from the group consisting of buprenorphine, fentanyl, pentazocine, tilidine, tramadol and/or naloxone.

22. The pharmaceutical composition according to one of the preceding claims, **characterised in that** the composition comprises as active substance at least one substance inhibiting the blood coagulation and/or a substance inhibiting the platelet aggregation which is selected form the group consisting of heparin, low molecular heparin, heparin-salts, preferably heparin-sodium and/or heparin-calcium, heparinoids and/or acetylsalicylic acid and/or esters thereof.

23. The pharmaceutical composition according to claim 22, **characterised in that** the composition comprises the heparin, low molecular heparin, heparin-salts, preferably heparin-sodium and/or heparin-calcium and/or the heparinoids in a concentration of between 250.000 I.E. and 2.500.000 I.E. per 100 g of the composition.

24. The pharmaceutical composition according to one of the claims 1 to 22, **characterised in that** the composition contains the heparin, the low molecular heparin, the heparin-salts, preferably the heparin-sodium and/or the heparin-calcium, and/or the heparinoids in a concentration of at least 20.000 I.E. for the application for sports injuries and accident injuries, bruises, varicose symptom complexes, superficial thromboses, thrombophlebitides, inflammable infiltrates, cicatricial hardening and/or haemorrhoids.

25. The pharmaceutical composition according to claim 23, **characterised in that** the composition contains the heparin, the low molecular heparin, the heparin-salts, preferably the heparin-sodium and/or the heparin-calcium, and/or the heparinoids in a concentration of between 120.000 I.E. and 240.000 I.E.

26. The pharmaceutical composition according to one of the preceding claims, **characterised in that** the composition comprises at least one corticoid active substance which is selected from the group consisting of dexamethasone, betamethasone, triamcinolone and/or clobetasole.

27. The pharmaceutical composition according to one of the preceding claims, **characterised in that** the composition comprises as active substance at least one histamine antagonist which is selected from the group consisting of bamipine lactate, chlorophenoxamine-HCl, clemastinhydrogenfumaric salt, dimetidindenmaleate, loratadine and/or pheniraminohydrogenmeleate.

28. The pharmaceutical composition according to one of the preceding claims, **characterised in that** the composition contains as active substance at least one anti-diabetic active substance, preferably insulin.

29. The pharmaceutical composition according to one of the preceding claims, **characterised in that** the composition contains as active substance at least one dermatologically effective substance.

30. The pharmaceutical composition according to one of the preceding claims, **characterised in that** the composition contains at least one local anaesthetic, preferably lidocaine-HCl, benzocaine and/or tetracaine.

31. The pharmaceutical composition according to one of the preceding claims, **characterised in that** the composition contains quinine and/or thalidomide as active substance.

32. The pharmaceutical composition according to one of the preceding claims, **characterised in that** the composition contains as active substance at least one chemotherapeutic, preferably an antibiotic, a cytostatic and/or at least one metastasis inhibitor.

33. The pharmaceutical composition according to one of the preceding claims, **characterised in that** the composition comprises as active substance at least one regulatory peptide and/or the inhibitors thereof which is, respectively are, selected from the group consisting of antuitary hormones and/or the inhibitors thereof, postpituitary hormones and/or the inhibitors thereof, hypothalamic hormones and/or the inhibitors thereof.

34. The pharmaceutical composition according to one of the preceding claims, **characterised in that** the composition comprises aciclovir as antiviral active substance.

35. The pharmaceutical composition according to one of the preceding claims, **characterised in that** the composition comprises as active substance prostaglandins, preferably PGE₁ as well as esters thereof, particularly the ethylester.

## Revendications

1. Composition pharmaceutique contenant au moins un agent à action systémique et / ou locale, applicable de manière topique, de préférence choisi dans le groupe formé par des anesthésiques locaux, des anti-allergiques, les agents dermatologiques, les agents contre les infections grippales et les pathologies de refroidissement, les agents pour le traitement de neuropathies, des agents chimiothérapeutiques, la quinine, la thalidomide, les analgésiques, des antirhumatismaux non stéroïdes, des agonistes des récepteurs opiacés, des antagonistes des récepteurs opiacés, des inhibiteurs de coagulation sanguine, des inhibiteurs d'agrégation de thrombocytes, des corticoïdes, des antagonistes de l'histamine, des antidiabétiques, des peptides régulateurs et leurs inhibiteurs, des prostaglandines et / ou leurs esters et / ou des agents actifs antiviraux, la composition présentant une consistance liquide telle qu'elle puisse être pulvérisée en gouttelettes sur une surface corporelle, **caractérisée en ce que** la composition liquide comporte un agent gélifiant à base d'un phospholipide ou d'un mélange de phospholipides, en une concentration de 0,5% en poids à 20% en poids, que la composition peut être pulvérisée en gouttelettes d'un diamètre de gouttelette de 10 µm à 100 µm et que la composition forme après pulvérisation, dans un très bref délai, de préférence dans un délai de moins de 4 secondes, sur la surface corporelle traitée, en particulier sur la peau ou la muqueuse vaporisée, une préparation dans laquelle l'agent actif est alors présent sous forme finement divisée et dans laquelle la concentration du au moins un agent actif est de 25% à 150% supérieure à la concentration du au moins un agent actif dans la composition liquide originelle avant la pulvérisation.

2. Composition pharmaceutique selon la revendication 1, **caractérisée en ce que** la composition forme la préparation dans un délai de moins de 2 secondes, de préférence dans le délai de 1 seconde à 0,01 seconde, sur la surface corporelle traitée.

3. Composition pharmaceutique selon la revendication 1 ou 2, **caractérisée en ce que** la composition présente, avant pulvérisation, une viscosité de 1 mPas à 100 mPas, de préférence de 5 mPas à 25 mPas.

4. Composition pharmaceutique selon l'une des revendications qui précèdent, **caractérisée en ce que** la composition ne contient, outre le au moins un agent actif et le gélifiant phospholipidique, qu'au moins un liquide ou un mélange de liquides, de sorte que la préparation formée sur la surface corporelle traitée consiste en le au moins un agent actif.

5. Composition pharmaceutique selon l'une des revendications qui précèdent, **caractérisée en ce que** la composition liquide est une dispersion, une solution et / ou une suspension et que la composition contient en tant que liquide ou mélange de liquides un solvant organique ou mélange de solvants organiques inoffensif et / ou de l'eau.

6. Composition pharmaceutique selon la revendication 4 ou 5, **caractérisée en ce que** le solvant organique comprend un alcool ou un mélange de solvants s'évaporant aisément.

7. Composition pharmaceutique selon l'une des revendications 4 à 6, **caractérisée en ce que** la concentration de l'alcool ou du mélange d'alcools dans la composition liquide varie de 5% en poids à 40% en poids, par rapport à la composition liquide.

8. Composition pharmaceutique selon l'une des revendications 4 à 7, **caractérisée en ce que** la composition liquide contient de 90% en poids à 50% en poids d'eau.

9. Composition pharmaceutique selon l'une des revendications qui précèdent, **caractérisée en ce que** la concentration du gélifiant phospholipidique dans la composition varie de 2% en poids à 14% en poids, par rapport à la composition liquide.

10. Composition pharmaceutique selon l'une des revendications qui précèdent, **caractérisée en ce que** le phospholipide ou mélange de phospholipides présent dans la composition en tant qu'agent gélifiant est un phospholipide ou un mélange de phospholipides isolé à partir de graines de soja, de tournesol et / ou d'oeuf.

11. Composition pharmaceutique selon l'une des revendications qui précèdent, **caractérisée en ce que** la composition comporte en tant que gélifiant un phospholipide ou un mélange de phospholipides contenant au moins 60% en poids de phosphatidylcholine, de préférence au moins 76% en poids de phosphatidylcholine et en particulier au moins 90% en poids de phosphatidylcholine.

12. Composition pharmaceutique selon l'une des revendications qui précèdent, **caractérisée en ce que** la composition comporte en tant que gélifiant un phospholipide ou un mélange de phospholipides contenant au moins 95% en poids de phosphatidylcholine.

13. Composition pharmaceutique selon l'une des revendications qui précèdent, **caractérisée en ce que** le mélange de phospholipides comporte au maximum 6% en poids de lysophosphatidylcholine, de préférence au maximum 4% en poids de lysophosphatidylcholine.

14. Composition pharmaceutique selon l'une des revendications 11 à 13, **caractérisée en ce que** la composition comporte une phosphatidylcholine dont les radicaux acyle en positions 1 et 2 sont constitués de 10 à 15% en poids du radical de l'acide palmitique, de 1,5 à 4% en poids du radical de l'acide stéarique, de 3 à 10% en poids du radical de l'acide oléique, de 61 à 71% en poids du radical de l'acide linoléique et de 3 à 7% en poids du radical de l'acide linolénique.

15. Composition pharmaceutique selon l'une des revendications qui précèdent, **caractérisée en ce que** la composition comporte en tant que gélifiant un phospholipide ou mélange de phospholipides hydrogéné.

16. Composition pharmaceutique selon la revendication 15, **caractérisée en ce que** la phosphatidylcholine contenue dans le phospholipide ou mélange de phospholipides hydrogéné contient, en positions 1 et 2, en tant que radical acyle, 85% en poids ± 3% en poids du radical de l'acide stéarique et 15% en poids ± 2% en poids du radical de l'acide palmitique.

17. Composition pharmaceutique selon l'une des revendications 13 à 19, **caractérisée en ce que** le mélange de phospholipides comporte, outre de la phosphatidylcholine en tant que principal constituant et le maximum de 6% en poids de lysophosphatidylcholine, de 0 à 12% en poids de phosphatidyléthanolamine, de 0 à 8% en poids de phosphatidylinositol et / ou de 0 à 8% en poids d'acide phosphoaminolipidique.

18. Composition pharmaceutique selon l'une des revendications qui précèdent, **caractérisée en ce que** la composition liquide est une dispersion et contient des liposomes.

19. Composition pharmaceutique selon l'une des revendications qui précèdent, **caractérisée en ce que** la composition contient l'agent actif à une concentration de 0,01% en poids à 20% en poids, de préférence à une concentration de 0,5% en poids à 10% en poids.

20. Composition pharmaceutique selon l'une des revendications qui précèdent, **caractérisée en ce que** la composition comporte au moins un agent actif analgésique et / ou antirhumatismal, choisi dans le groupe formé par l'Acemetacin, le Diclofenac et ses sels, de préférence le Diclofenac sodique et / ou le Diclofenac - diéthylamine, l'Etofenamat, le Flufenaminoacide, l'Ibuprofen, des formes racémiques et / ou des énantiomères d'Ibuprofen, de préférence le S(+)-Ibuprofen, l'Indometacin, le kétoprofène, des formes racémiques et / ou des énantiomères de kétoprofène, le piroxicam, l'acide salicylique et / ou ses dérivés, de préférence l'acide acétylsalicylique et / ou l'acide 2-hydroxyéthylsalicylique et / ou des antirhumatismaux / antiallergiques non stéroïdes sélectifs de la cyclooxygénase-2.

21. Composition pharmaceutique selon l'une des revendications qui précèdent, **caractérisée en ce que** la composition comporte en tant qu'agent actif au moins un agoniste des récepteurs opiacés et / ou antagoniste des récepteurs opiacés, choisi dans le groupe formé par la buprénorphine, le fentanyl, la pentazocine, la péthidine, la tilidine, le Tramadol et / ou le Naxolon.

22. Composition pharmaceutique selon l'une des revendications qui précèdent, **caractérisée en ce que** la composition comporte comme agent actif au moins un agent inhibant la coagulation sanguine et / ou un agent inhibant l'agrégation des thrombocytes, choisi dans le groupe formé par l'héparine, l'héparine à bas poids moléculaire, des sels d'héparine, de préférence de l'héparine sodique et / ou de l'héparine calcique, des héparinoïdes et / ou de l'acide acétylsalicylique et / ou ses esters.

23. Composition pharmaceutique selon la revendication 22, **caractérisée en ce que**, dans la composition, l'héparine, l'héparine à bas poids moléculaire, les sels d'héparine, de préférence de l'héparine sodique et / ou de l'héparine calcique, et / ou les héparinoïdes, sont contenus à une concentration de 250.000 I.E. à 2.500.000 I.E. par 100 g de composition.

24. Composition pharmaceutique selon l'une des revendications 1 à 22, **caractérisée en ce que** la composition contient, pour l'application locale en cas de lésions sportives ou accidentelles, épanchements sanguins, symptômes variqueux complexes, infiltrations inflammatoires, indurations cicatricielles et / ou hémorroïdes, l'héparine, l'héparine à bas poids moléculaire, les sels d'héparine, de préférence de l'héparine sodique et / ou de l'héparine calcique, et / ou les héparinoïdes à une concentration d'au moins 20.000 I.E.

25. Composition pharmaceutique selon la revendication 23, **caractérisée en ce que** la composition contient l'héparine, l'héparine à bas poids moléculaire, les sels d'héparine, de préférence de l'héparine sodique et / ou de l'héparine calcique, et / ou les héparinoïdes en une concentration de 120.000 I.E. à 240.000 I.E.

26. Composition pharmaceutique selon l'une des revendications qui précèdent, **caractérisée en ce que** la composition comporte au moins un agent actif corticoïde, choisi dans le groupe formé par la dexaméthasone, la bétaméthasone, la triamcinolone et / ou le clobétasol.

27. Composition pharmaceutique selon l'une des revendications qui précèdent, **caractérisée en ce que** la composition contient en tant qu'agent actif au moins un antagoniste de l'histamine, choisi dans le groupe formé par le lactate de bamipine, la Chlorophénoxamine- HCl, l'hydrogénofumarate de clémastine, l'indène maléate de dimétidine, la loratadine et / ou l'hydrogénomaléate de phéniramine.

28. Composition pharmaceutique selon l'une des revendications qui précèdent, **caractérisée en ce que** la composition contient en tant qu'agent actif au moins un agent actif antidiabétique, de préférence une insuline.

29. Composition pharmaceutique selon l'une des revendications qui précèdent, **caractérisée en ce que** la composition contient en tant qu'agent actif au moins un agent dermatologiquement actif.

30. Composition pharmaceutique selon l'une des revendications qui précèdent, **caractérisée en ce que** la composition contient au moins un anesthésique local, de préférence de la lidocaïne - HCl, de la benzocaïne et / ou de la tétracaïne.

31. Composition pharmaceutique selon l'une des revendications qui précèdent, **caractérisée en ce que** la composition contient en tant qu'agent actif de la quinine et / ou de la thalidomide.

32. Composition pharmaceutique selon l'une des revendications qui précèdent, **caractérisée en ce que** la composition contient en tant qu'agent actif au moins un agent chimiothérapeutique, de préférence un antibiotique, un cytostatique et/ou au moins un inhibiteur de métastases.

33. Composition pharmaceutique selon l'une des revendications qui précèdent, **caractérisée en ce que** la composition contient en tant qu'agent actif au moins un peptide régulateur et / ou ses inhibiteurs, choisi(s) par exemple dans le groupe formé par des hormones du lobe antérieur de l'hypophyse et / ou leurs inhibiteurs, des hormones du lobe postérieur de l'hypophyse et / ou leurs inhibiteurs, des hormones hypothalamiques et / ou leurs inhibiteurs.

34. Composition pharmaceutique selon l'une des revendications qui précèdent, **caractérisée en ce que** la composition comporte de l'aciclovir en tant qu'agent antiviral.

35. Composition pharmaceutique selon l'une des revendications qui précèdent, **caractérisée en ce que** la composition comporte en tant qu'agent actif de la prostaglandine, de préférence PGE₁, ainsi que ses esters, en particulier l'éthylester.
